**Europäisches Patentamt**

**European Patent Office**

**Office européen des brevets**

(19)

(11) Veröffentlichungsnummer: **0 019 042**
**B1**

(12) # EUROPÄISCHE PATENTSCHRIFT

(45) Veröffentlichungstag der Patentschrift:
27.07.83

(51) Int. Cl.³: **A 61 F 1/03**

(21) Anmeldenummer: **80101207.1**

(22) Anmeldetag: **10.03.80**

(54) **Oberschenkelteil einer Hüftgelenkendoprothese.**

(30) Priorität: **19.05.79 DE 2920354**

(43) Veröffentlichungstag der Anmeldung:
**26.11.80 Patentblatt 80/24**

(45) Bekanntmachung des Hinweises auf die Patenterteilung:
**27.07.83 Patentblatt 83/30**

(84) Benannte Vertragsstaaten:
**AT CH FR GB**

(56) Entgegenhaltungen:
**AT-A-351 152**
**DE-A-2 142 820**
**DE-B-2 621 384**
**FR-A-2 360 295**

(73) Patentinhaber: **SIGRI ELEKTROGRAPHIT GMBH,**
**Werner von Siemens-Strasse 18, D-8901 Meitingen (DE)**

(72) Erfinder: **Gerstenberger, Friedrich, Prof. Dipl.-Ing.,**
**Grabenstrasse 11, D-7321 Dürnau (DE)**
Erfinder: **Loos, Wolfgang, Ing. grad., Studentenweg 6,**
**D-7932 Munderkingen (DE)**

## Oberschenkelteil einer Hüftgelenkendoprothese

Die Erfindung betrifft den Oberschenkelteil einer Hüftgelenkendoprothese, mit einem ein Gewinde aufweisenden, aus mit Kohlenstoffasern verstärktem Kohlenstoff bestehenden Prothesenschaft und einer mit dem Schaft lösbar verbundenen Gelenkkugel.

Als Ersatz für funktionsuntüchtig gewordene Gelenke, besonders für Hüftgelenke, sind Endoprothesen aus einer Vielzahl von Werkstoffen vorgeschlagen worden, die in der Regel mit Knochenzement in einer Ausnehmung des Knochens, z. B. des Femurs, verankert sind. Diese Art der Verankerung befriedigt nicht immer, da Volumenänderungen des überwiegend aus einem Kunstharz bestehenden Knochenzements während der Härtung des Harzes, Alterungsvorgänge im Harz, die verschiedene Elastizität oder Steifigkeit von Knochen und Prothesenschaft und die unphysiologische Krafteinleitung in das körpereigene Knochengewebe nach einem mehr oder weniger kurzen Zeitabschnitt zu einer Lockerung der Prothese führen. Es ist auch bekannt, den stielartigen Schaft einer Hüftgelenkendoprothese mit einem Gewinde zu versehen und in eine ein Gewinde aufweisende Ausnehmung des Femurs zu schrauben. Die Verwendung eines besonderen Knochenzements ist bei dieser Ausführung nicht nötig. Durch die Verwendung von Werkstoffen für den Prothesenschaft, welche die gleiche oder wenigstens eine angenähert gleiche Elastizität wie das Knochengewebe aufweisen, kann darüber hinaus die unphysiologische Dehnungshinderung des Knochens vermindert werden. Aus Kohlenstoff bestehende Prothesenschäfte (DE-A-2 142 820) und besonders Schäfte aus mit Kohlenstoffasern verstärktem Kohlenstoff erfüllen diese Bedingung (FR-A-2 360 295). Die übereinstimmende oder nahezu übereinstimmende Elastizität von Knochen und Prothese ermöglicht eine homogene Krafteinleitung und schließt überkritische Belastungen einzelner Teile der Verbindung aus. Die zementfreie Verankerung der Prothese, die Verwendung isoelastischer Werkstoffe und die Kombination dieser Maßnahmen genügen jedoch nicht, den strukturellen Umbau des Knochengewebes nach dem Wolffschen Gesetz und die dadurch bedingte allmähliche Lockerung der Prothese zu verhindern. Die strukturelle Umbildung oder Rückbildung des Knochengewebes wird besonders durch schwellende Zugspannungen und Mikrobewegungen im Bereich der Gewindeauflageflächen und auch durch Zugspannungen am medialen, proximalen Ende des Femurs verursacht. Eine weitere Ursache der ebenfalls unbefriedigenden Lebensdauer dieser Prothesenverankerungen ist die große Variabilität der Knochenelastizität, so daß die Elastizitätsmoduln von Knochen und Prothesenwerkstoff höchstens zufällig übereinstimmen. Bei verscheidenen Moduln ist aber der Spannungsaufbau in der Verbindung nicht konstant und es resultiert eine inkonstante Spannungsverteilung über die Gangbreite des Gewindes. Die bekannte Gewindeform für Kortikalis- und Spongiaschrauben nach DIN 58 810 löst zudem in der Schraubverbindung hohe besonders bei Dauerbelastungen unter schwellenden Lasten nachteilige Kerbspannungsspitzen aus.

Der Erfindung liegt daher die Aufgabe zugrunde, die Mikrobewegungen zwischen Knochengewebe und Prothese insbesondere im Bereich der Gewindeauflageflächen und den durch inhomogene Spannungsverteilung verursachten differentiellen Umbau des Knochengewebes und damit eine Lockerung der Prothese zu vermeiden und die Lebensdauer der Prothesenverankerung zu verlängern.

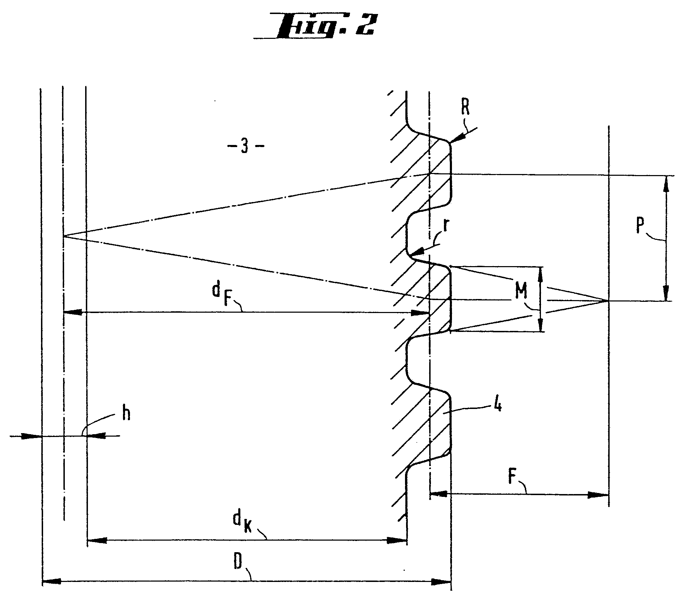

Die Aufgabe wird mit einer Prothese der eingangs genannten Art gelöst, deren Schaft einen Elastizitätsmodul aufweist, der zwei- bis dreimal größer als der Modul des Femurknochens ist, deren Gewinde ein Trapezgewinde ist, bei dem das Verhältnis der Gewindesteigung $P$ zur Gewindekopfbreite $M$ $(P-M)/h$ 0,8 bis 1,2 beträgt, wobei die Gewindekopfbreite $M$ die Länge des parallel zur Schraubenachse verlaufenden Abschnitts des Gewindekopfs ist, und der von zwei unmittelbar gegenüberliegenden Flanken aufgespannte Flankenwinkel $\alpha$ kleiner als 15° ist. Die Begriffe $P$ und $M$ sind in der Figur 2 erläutert. Unter dem Flankenwinkel $\alpha$ ist der von zwei unmittelbar gegenüberliegenden Flanken bzw. deren Wendegraden aufgespannte Winkel zu verstehen (G. Niehaus, Maschinenelemente, Bd. 1, Springer Verlag 1955, Seiten 160 bis 162 und Tafel 10/1). Nach einer bevorzugten Ausbildung der Erfindung ist das Verhältnis von Gewindehöhe $h$ zur Gewindekopfbreite $M$ kleiner als eins. Als Werkstoff für den Prothesenschaft eignet sich besonders mit Kohlenstoffasern verstärkter Kohlenstoff.

Die erfindungsgemäße Prothese ermöglicht es, permanente Vorspannungen auf den mit dem Prothesenschaft verschraubten Femur aufzubringen. Ein wesentlicher Vorteil der neuen Prothesenverankerung besteht darin, daß die eingebrachte Vorspannkraft der Schraube bei überlagernd auftretenden dynamischen Beanspruchungen kein Abheben der Trennfuge, keine merklichen plastischen Deformationsverschiebungen und keine Zugdehnungen im Knochen zuläßt. Bei der Verwendung von Prothesenschäften aus kohlenstoffaserverstärktem Kohlenstoff (CFC) kommt als weiterer Vorteil die stärkere Belastung der Gewindegänge aus Kohlenstoff durch Biegespannungen und die stärkere Belastung des Knochens durch Scherspannungen zum Tragen in Übereinstimmung und unter Ausnutzung der verschiedenen Festigkeitswerte dieser Stoffe, die beispielsweise

## 0 019 042

|  | Knochen | CFC |
|---|---|---|
| Biegefestigkeit | 80 | 500 N/mm$^2$ |
| Scherfestigkeit | 90 | 20 N/mm$^2$ |

betragen. In dem neuen Prothesengewinde wird der Werkstoff mit der geringen Scherfestigkeit bevorzugt an der Gewindespitze belastet und der biegeschwächere, eine größere Scherfestigkeit aufweisende Knochen wird mehr zum Gewindegrund hin beansprucht. Schließlich werden die durch unterschiedliche Gangbelastungen bedingte auf den Knochen wirkende unphysiologischen Radialspannungen, die bei den bekannten Schaftgewinden von Hüftgelenkendoprothesen vermutlich die großen Schwierigkeiten bereiten, wesentlich reduziert.

Im folgenden wird die Erfindung anhand von Zeichnungen beispielshaft erläutert:

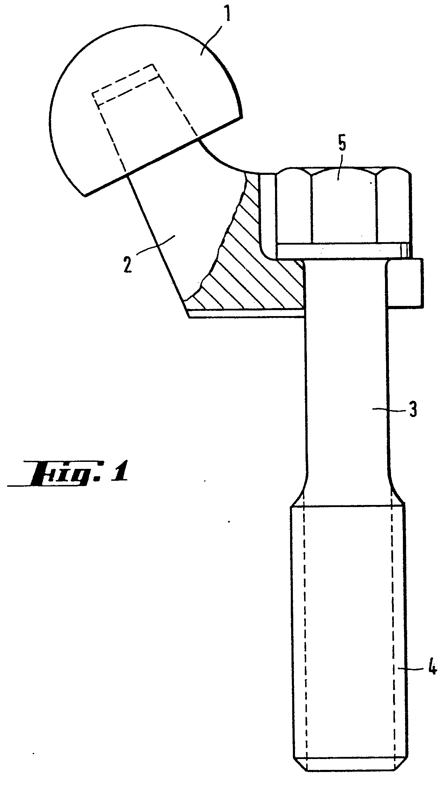

Fig. 1 zeigt in einem Schnitt den Oberschenkelteil einer Hüftgelenkendoprothese, deren Schaft über einen Teil ihrer Länge mit einem Gewinde versehen ist, und

Fig. 2 zeigt einen Schnitt durch einen Teil des Prothesenschafts.

Der Gelenkkopf 1, der beispielsweise aus gesintertem Aluminiumoxid oder einem anderen abriebfesten keramischen Stoff besteht, ist auf den an seinem oberen Ende konisch ausgebildeten kragenförmigen Prothesenteil 2 axial aufgeschoben. Dieser eine schwalbenschwanzförmige oder kreisförmige Ausnehmung aufweisende Teil 2 ist auf den Prothesenschaft 3 geschoben und mit diesem durch den Schraubenkopf 5 kraft- oder formschlüssig verbunden. Der untere Teil des Prothesenschafts weist ein Trapezgewinde 4 auf, und greift in eine zeichnerisch nicht dargestellte mit einem Gegengewinde versehene Ausnehmung des Femurs ein.

Wenigstens der Prothesenschaft, zweckmäßig auch der kragenförmige Teil der Prothese bestehen aus mit Kohlenstoffasern verstärktem Kohlenstoff, dessen Elastizitätsmodul zwei- bis dreimal größer als der Modul des Knochens ist. Unter diesem Begriff sind Verbundwerkstoffe zu verstehen, die als Matrix carbonisierte Duro- oder Thermoplaste oder auch Steinkohlenteerpeche und als Verstärkungselemente Kohlenstoff- oder Graphitfasern in Form von Filamenten, Kurzschnittfasern, Matten und anderen textilen Flächengebilden enthalten und eine vorzügliche Gewebeverträglichkeit aufweisen.

In der Darstellung nach Fig. 2 sind

D = Außendurchmesser, $d_k$ = Kerndurchmesser,
P = Steigung, M = Gewindekopfbreite und $\alpha$ = Flankenwinkel,
R, r = Krümmungsradien des Gewindes, das folgenden Bedingungen genügt:

1. $\dfrac{P-M}{M} = 0{,}8 - 1{,}2,$

2. $\alpha < 15°$ und vorzugsweise

3. $\dfrac{D-d_k}{M} = \dfrac{h}{M} < 1.$

Es folgen zwangsläufig einige andere Beziehungen, z. B.

4. $\dfrac{P}{\dfrac{M+h \cdot tg\,\alpha}{2 \cdot tg\,\alpha}} = \dfrac{P}{F} = 0{,}6 - 0{,}8.$

Die Auslegung des Gewindes in Abhängigkeit vom Außendurchmesser D ist in der Tabelle beispielhaft dargestellt.

| D | $d_k$ | h | P | M | $\dfrac{P-M}{M}$ | h/M | P/F |
|---|---|---|---|---|---|---|---|
| 24 mm | 18,5 mm | 2,75 mm | 6 mm | 2,93 mm | 1,05 | 0,94 | 0,71 |
| 20 | 15,4 | 2,3 | 5 | 2,45 | 1,04 | 0,94 | 0,70 |
| 16 | 11,4 | 2,3 | 5 | 2,45 | 1,04 | 0,94 | 0,70 |
| 10 | 7,8 | 1,1 | 3 | 1,52 | 0,97 | 0,72 | 0,70 |

**0 019 042**

Der Flankenwinkel $\alpha$ betrug bei diesem Beispiel 11,6°. Zur Vermeidung von Kerbspannungen im Gewindegrund sollten die Krümmungsradien R − 0,5 bis 1,10 und r − 0,3 bis 0,8 betragen.

**Patentansprüche**

1. Oberschenkelteil einer Hüftgelenkendoprothese mit einem ein Gewinde aufweisenden, aus mit Kohlenstoffasern verstärktem Kohlenstoff bestehenden Prothesenschaft und einer mit dem Schaft lösbar verbundenen Gelenkkugel, dadurch gekennzeichnet, daß

a) der Elastizitätsmodul des Schafts zwei- bis dreimal größer als der Elastizitätsmodul des Femurknochens ist,
b) das Gewinde ein Trapezgewinde ist, bei dem das Verhältnis der Gewindesteigung P zur Gewindekopfbreite M (P-M)/M 0,8 bis 1,2 beträgt, wobei die Gewindekopfbreite M die Länge des parallel zur Schraubenachse verlaufenden Abschnitts des Gewindekopfs ist, und
c) der von zwei unmittelbar gegenüberliegenden Flanken aufgespannte Flankenwinkel $\alpha$ kleiner als 15° ist.

2. Prothese nach Patentanspruch 1, dadurch gekennzeichnet, daß das Verhältnis der Gewindehöhe h, worunter die Differenz von Außendurchmesser und Kerndurchmesser zu verstehen ist, zur Gewindekopfbreite M kleiner als 1 ist.

3. Prothese nach den Patentansprüchen 1 und 2, dadurch gekennzeichnet, daß auf dem mit dem Femur verschraubten Prothesenschaft eine Druckvorspannung aufgebracht ist.

**Claims**

1. Femur part of a hip joint endoprosthesis with a prosthesis shaft consisting of carbon reinforced with carbon fibres, possessing a screw threading, and with a coupling ball releasably connected with the shaft, characterised in that

a) the elasticity modulus of the shaft is two to three times greater than the elasticity modulus of the femur bone,
b) the screw-threading is a trapezoidal threading in which the ratio of the screw pitch P to the thread head width M (P-M)/M amounts to 0.8 to 1.2, with the thread head width M being the length of the segment of the thread head running parallel to the screw axis, and
c) the included angle of thread set by two flanks lying directly opposite one another is less than 15°.

2. Prosthesis according to claim 1, characterised in that the ratio of the thread height h by which is to be understood the difference between the outer diameter and the core diameter, to the thread head width M is less than 1.

3. Prosthesis according to claims 1 and 2, characterised in that a compressive stress is applied to the prosthesis shaft screwed with the femur.

**Revendications**

1. Partie de haut de cuisse d'une endoprothèse de l'articulation de la hanche avec une tige de prothèse présentant un filetage, constituée en carbone renforcé avec des fibres de carbone et une articulation sphérique assemblée de façon amovible avec la tige, caractérisée en ce que:

a) le module d'élasticité de la tige est de de deux à trois fois plus élevé que le module d'élasticité de l'os du fémur,
b) le filetage est un filetage trapézoïdal, dans lequel le rapport du pas P du filetage à la largeur de tête M des filets est tel que (P-M)/M, soit compris entre 0,8 à 1,2; la largeur de tête M des filets étant la longueur de la partie de la tête des filets s'étendant parallèlement à l'axe de la vis et
c) l'angle de flancs $\alpha$, fait par deux flancs directement opposés, est inférieur à 15°.

2. Prothèse selon revendication 1, caractérisé en ce que le rapport de la hauteur h du filetage, définie en tant que différence entre le diamètre extérieur et le diamètre du noyau, à la largeur de tête M des filets, est inférieur à 1.

3. Prothèse selon les revendications 1 et 2, caractérisée en ce qu'une précontrainte de compression est appliquée à la tige de prothèse vissée avec le fémur.

4

*Fig. 1*

# Fig. 2